# FASCICULE DE BREVET EUROPEEN

(11) **EP 2 336 745 B1**
(45) Date de publication et mention de la délivrance du brevet: **31.08.2016**
(21) Numéro de dépôt: 10195714.0
(22) Date de dépôt: 17.12.2010
(51) Int. Cl.: G01N 1/28, G01N 13/00, G01N 33/20, G01N 1/42, G01N 1/44

(54) **Procédé pour la préparation d'échantillons métalliques et autoclave pour la mise en oeuvre du procédé**
Verfahren zur Vorbereitung metallischer Proben und Autoklav zur Umsetzung dieses Verfahrens
Method for the preparation of metallic samples and autoclave for realisation of that method

(30) Priorité: 18.12.2009 FR 0959243
(43) Date de publication de la demande: 22.06.2011
(73) Titulaire: Commissariat à l'Énergie Atomique et aux Énergies Alternatives, 75015 Paris (FR)
(72) Inventeur: Datcharry, Frédéric, 78120, Rambouillet (FR); Marchetti, Loïc, 91310, Leuville-sur-orge (FR); Jambon, Fanny, 95100, Argenteuil (FR)
(74) Mandataire: Regimbeau

(56) Documents cités:
- FR-A- 1 435 934
- US-A- 4 192 175
- WAGNER J A ET AL: "Hydrogen effects on the age hardening behavior of 2024 aluminum", SCRIPTA METALLURGICA, ELSEVIER SCIENCE LTD., KIDLINGTON, GB LNKD- DOI:10.1016/0036-9748(86)90416-3, vol. 20, no. 7, 1 juillet 1986 (1986-07-01), pages 957-960, XP022827840, ISSN: 0036-9748 [extrait le 1986-07-01]
- WANG X L ET AL: "Reaction kinetics of hydrogen-metal hydride systems", INTERNATIONAL JOURNAL OF HYDROGEN ENERGY, ELSEVIER SCIENCE PUBLISHERS B.V., BARKING, GB LNKD- DOI:10.1016/0360-3199(80)90005-1, vol. 15, no. 8, 1 janvier 1990 (1990-01-01), pages 569-577, XP025640095, ISSN: 0360-3199 [extrait le 1990-01-01]
- YAO M Y ET AL: "The effect of alloying modifications on hydrogen uptake of zirconium-alloy welding specimens during corrosion tests", JOURNAL OF NUCLEAR MATERIALS, ELSEVIER BV, NL LNKD- DOI:10.1016/J.JNUCMAT.2005.12.005, vol. 350, no. 2, 15 avril 2006 (2006-04-15), pages 195-201, XP025187847, ISSN: 0022-3115 [extrait le 2006-04-15]
- RENAUDIN G ET AL: "Structural study of metal-hydrogen interactions in cubic PrH2+x and rare-earth analogues", JOURNAL OF ALLOYS AND COMPOUNDS, ELSEVIER SEQUOIA, LAUSANNE, CH LNKD- DOI:10.1016/S0925-8388(01)01497-9, vol. 330-332, 17 janvier 2002 (2002-01-17) , pages 175-178, XP004313254, ISSN: 0925-8388
- BROUWER ET AL: "Hydrogen diffusion and solubility in vanadium modified pressure vessel steels", SCRIPTA METALLURGICA ET MATERIALIA, OXFORD, GB LNKD- DOI:10.1016/0956-716X(92)90525-J, vol. 27, no. 3, 1 août 1992 (1992-08-01), pages 353-358, XP024182062, ISSN: 0956-716X [extrait le 1992-08-01]
- LANZANI L ET AL: "Comments on the stability of zirconium hydride phases in Zircaloy", JOURNAL OF NUCLEAR MATERIALS, ELSEVIER BV, NL LNKD- DOI:10.1016/J.JNUCMAT.2003.09.013, vol. 324, no. 2-3, 15 janvier 2004 (2004-01-15), pages 165-176, XP004480866, ISSN: 0022-3115

## Description

### Domaine de l'invention

L'invention concerne un procédé de préparation d'échantillons pour l'étude des interactions de matériaux avec de l'hydrogène lors de leur contact avec un liquide aqueux à haute température contenant de l'hydrogène dans un autoclave.

L'invention concerne également un autoclave pour la mise en oeuvre du procédé.

### État de la technique

L'hydrogène présent dans les liquides aqueux est connu pour interagir avec des matériaux métalliques et pour induire, dans certains cas, une dégradation prématurée de structures composées des matériaux, comme suite à une absorption de l'hydrogène par les matériaux. Cette dégradation prématurée est appelée fragilisation par l'hydrogène (FPH)

La FPH est un des mécanismes suspectés dans la dégradation de composants (partiellement ou entièrement en matériaux métalliques) des réacteurs nucléaires de deuxième et troisième générations.

L'étude de la FPH consiste à faire interagir un échantillon métallique solide avec l'hydrogène alors qu'il est exposé à un mélange aqueux (pouvant contenir de l'hydrogène dissous) porté à haute température (325 °C à 360 °C) dans une chambre d'un autoclave pendant une durée allant de quelques minutes à plusieurs milliers d'heure.

Afin d'étudier la FPH sur des matériaux utilisés dans des composants destinés à être au contact d'un liquide aqueux à haute température dans un réacteur nucléaire, le procédé connu suivant est habituellement mis en oeuvre.

Un échantillon de matériau est disposé dans un autoclave, au contact du mélange aqueux à haute température contenant de l'hydrogène pendant plusieurs heures, voire plusieurs jours, sous une pression comprise entre 110 et 200 bars.

Pendant la durée de mise en contact de l'échantillon avec le mélange aqueux, l'hydrogène contenu dans le mélange aqueux est absorbé par le matériau constituant l'échantillon. C'est-à-dire que les atomes d'hydrogène s'immiscent dans le réseau cristallin du matériau constituant l'échantillon et interagissent avec la microstructure du matériau, parfois en la fragilisant : c'est la FPH.

A la fin de la durée de mise en contact avec le mélange aqueux à haute température, l'échantillon est refroidi pendant plusieurs heures, jusqu'à ce qu'il atteigne une température d'environ 50 C facilitant ainsi sa manipulation et son analyse.

Or, la durée de refroidissement est relativement longue, essentiellement à cause du volume et de la masse importants de l'autoclave, ce qui fait que l'hydrogène absorbé par le matériau a le temps de désorber, car l'hydrogène présente une forte diffusivité.

C'est pourquoi, en général, l'échantillon analysé n'est pas représentatif de l'état du matériau lors de la mise au contact avec le liquide aqueux à haute température. Wagner et al. (Scripta Metallurgica, Volume 20, Issue 7, 1986, pages 957-960) divulgue un procédé de préparation d'un échantillon métallique introduit dans une chambre d'exposition d'un autoclave pour des interactions du matériau composant l'échantillon avec de l'hydrogène d'un mélange aqueux qui comprend une étape de refroidissement en dehors de l'autoclave. Wang et al. (International Journal of Hydrogen Energy, Volume 15, No. 8, 1990, pages 569 - 577) divulgue une étude de la cinétique des interactions entre hydrogène et un métal. Dans cette divulgation il y a une injection d'un gaz sous une haute dans les tuyaux d'échange thermique d'un autoclave.

### Présentation de l'invention

L'objectif de l'invention est de pallier au moins un des inconvénients ci-dessus.

Pour cela, on propose selon l'invention un procédé de préparation d'un échantillon métallique introduit dans une chambre d'exposition d'un autoclave, l'échantillon étant destiné à être analysé pour l'étude des interactions du matériau composant l'échantillon avec de l'hydrogène d'un mélange aqueux à haute température auquel l'échantillon est exposé dans la chambre, caractérisé en ce qu'il comprend une étape de refroidissement par injection de liquide de refroidissement sous une haute pression supérieure à 50 bars dans la chambre d'exposition de l'autoclave par l'intermédiaire d'un injecteur, pour refroidir l'échantillon et la chambre d'exposition et ainsi éviter une évaporation du liquide de refroidissement sous haute pression lors de son contact avec l'échantillon et/ou la chambre d'exposition.

D'autres caractéristiques optionnelles et non limitatives sont :
- le liquide de refroidissement sous haute pression est injecté sous une pression supérieure à 70 bars ;
- le procédé comprend en outre une étape d'ouverture d'un échappement de l'autoclave, antérieure à l'étape de refroidissement par injection de liquide de refroidissement sous haute pression, pour l'évacuation du liquide aqueux du mélange hors de la chambre, provoquant une détente de la vapeur du mélange à l'intérieur de la chambre de l'autoclave et ainsi refroidissant l'échantillon et/ou la chambre de l'autoclave ;
- le procédé comprend en outre l'étape d'injection de liquide de refroidissement sous une basse pression comprise entre 2 et 11 bars dans l'autoclave pour maintenir relativement constante la température de l'échantillon et continuer le refroidissement de la chambre de l'autoclave, l'étape d'injection de liquide de refroidissement sous basse pression étant postérieure à l'étape d'injection de liquide de refroidissement sous haute pression ; et
- l'étape d'injection de liquide de refroidissement sous haute pression est arrêtée avant l'étape d'injection de liquide de refroidissement sous basse pression.

On propose également selon l'invention un autoclave pour la mise en oeuvre du procédé selon l'un des exemples de l'invention, comprenant une chambre d'exposition pour recevoir un mélange aqueux à haute température et un échantillon destiné à être analysé pour l'étude des interactions du matériau composant l'échantillon avec de l'hydrogène du mélange aqueux à haute température ;
caractérisé en ce qu'il comprend en outre un injecteur haute pression pour l'injection de liquide de refroidissement sous une haute pression supérieure à 50 bars.

D'autres caractéristiques optionnelles et non limitatives sont :
- l'injecteur haute pression comprend un clapet anti-retour haute pression ouvrable pour faire passer le liquide de refroidissement sous haute pression dans la chambre quand la chambre est sous une pression inférieure à la haute pression ;
- l'autoclave comprend un échappement ouvrable pour l'évacuation du liquide aqueux du mélange hors de la chambre, provoquant une détente de la vapeur du mélange à l'intérieur de la chambre de l'autoclave ;
- l'autoclave comprend, en outre, un injecteur basse pression pour l'injection de liquide de refroidissement sous une basse pression comprise entre 2 et 11 bars dans la chambre de l'autoclave ; et
- l'injecteur basse pression comprend un clapet anti-retour basse pression ouvrable pour faire passer le liquide de refroidissement sous basse pression dans la chambre d'exposition quand la chambre d'exposition est sous une pression inférieure à la basse pression.

Un avantage du procédé et de l'autoclave selon l'invention est de permettre l'étude de la FPH de matériaux métalliques exposés dans un liquide aqueux à haute température pendant une durée de plusieurs heures, voire plusieurs jours, en fournissant des échantillons représentatifs de l'état des matériaux métalliques à l'intérieur de l'autoclave lorsqu'ils sont au contact avec le liquide aqueux à haute température.

En effet, les conditions de refroidissement utilisées permettent d'éviter que l'hydrogène absorbé dans l'échantillon ne désorbe.

Un avantage de l'autoclave selon l'invention est qu'il est moins volumineux et plus facilement maniable que les autoclaves connus de l'état de l'art.

### Présentation des figures

D'autres caractéristiques, buts et avantages de l'invention apparaîtront à la lecture de la description détaillée qui suit en référence aux dessins donnés à titre illustratif et non limitatif, parmi lesquels :
- la figure 1 est un organigramme montrant la succession des étapes du procédé selon un exemple de l'invention ;
- la figure 2 est un schéma d'un autoclave pour la mise en oeuvre du procédé selon un exemple de l'invention ; et
- la figure 3 est un graphe montrant des résultats d'un essai avec un exemple d'autoclave selon l'invention mettant en oeuvre un exemple de procédé selon l'invention.

### Description détaillé de l'invention

### Procédé

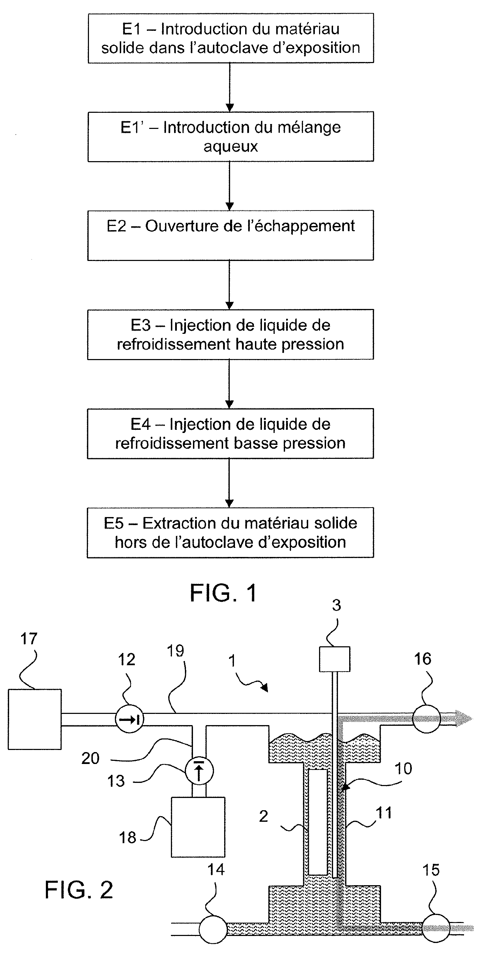

Un procédé de préparation d'un échantillon métallique selon l'invention est décrit par la suite en référence aux figures 1 et 2.

L'échantillon **2,** introduit dans une chambre **10** d'un autoclave **1,** est destiné à être analysé pour l'étude des interactions du matériau composant l'échantillon **2** avec de l'hydrogène d'un mélange aqueux à haute température auquel l'échantillon **2** est exposé dans la chambre.

Ce procédé comprend une étape de refroidissement par injection **E3** de liquide de refroidissement sous une haute pression dans la chambre **10** de l'autoclave **1** par l'intermédiaire d'un injecteur **17.** L'injection **E3** de liquide de refroidissement sous haute pression permet de refroidir l'échantillon **2** et la chambre **10,** tout en évitant une évaporation du liquide de refroidissement sous haute pression lors de son contact avec l'échantillon **2** et la chambre **10.**

La haute pression est choisie supérieure à 50 bars. De préférence, la haute pression est choisie supérieure à 70 bars.

Le procédé comprend principalement les étapes de :
- introduction **E1** de l'échantillon **2** à l'intérieur de la chambre **10** de l'autoclave **1 ;**
- introduction **E1'** d'un mélange aqueux ;
- éventuellement ouverture **E2** d'un échappement **14** de l'autoclave **1 ;**
- injection **E3** de liquide de refroidissement sous haute pression ;
- éventuellement, d'injection **E4** de liquide de refroidissement sous une basse pression comprise entre 2 et 11 bars dans la chambre **10** de l'autoclave **1 ;** et
- extraction **E5** de l'échantillon **2** hors de la chambre **10** de l'autoclave **1** pour son analyse.

Le remplissage de la chambre **10** avec le mélange aqueux est assuré par un circuit de recirculation de l'autoclave **1** comprenant une vanne d'entrée **15** et une vanne de sortie **16.** Les vannes d'entrée **15** et de sortie **16** sont ouvertes pour le remplissage de la chambre **10** et pendant la durée (pouvant aller jusqu'à plusieurs jours) d'exposition de l'échantillon au mélange aqueux haute température. Ces vannes d'entrée **15** et de sortie **16** sont fermées à la fin de l'exposition de l'échantillon **2** au mélange aqueux.

L'ouverture **E2** de l'échappement **14** permet l'évacuation du liquide aqueux du mélange hors de la chambre **10 ;** ceci provoque une détente de la vapeur du mélange à l'intérieur de la chambre **10** de l'autoclave **1** et l'échantillon **2** et/ou la chambre **10** de l'autoclave **1** est/sont refroidi(e/s). Cette étape d'ouverture **E2** de l'échappement est antérieure à l'étape d'injection **E3** de liquide de refroidissement sous haute pression.

L'injection **E4** de liquide de refroidissement sous basse pression permet de maintenir relativement constante la température de l'échantillon **2** tout en continuant le refroidissement de la chambre **10** de l'autoclave **1.** Cette étape d'injection **E4** de liquide de refroidissement sous basse pression est postérieure à l'étape d'injection **E3** de liquide de refroidissement sous haute pression.

Dans ce dernier cas, l'étape d'injection **E3** de liquide de refroidissement sous haute pression peut être arrêtée avant l'étape d'injection **E4** de liquide de refroidissement sous basse pression.

L'intégration de clapets anti-retour **12, 13** au niveau, respectivement, des injecteurs haute pression **17** et basse pression **18** constitue une optimisation du procédé. En effet, l'utilisation de clapets anti-retour **12, 13** permet une gestion mécanique, par différence de pression, des ouvertures et fermetures en série des injecteurs haute pression **17** et basse pression **18.** L'élimination de l'intervention humaine, dans la gestion des ouvertures et fermetures des injecteurs haute pression **17** et basse pression **18,** permet d'augmenter la vitesse de refroidissement de l'échantillon **2** et/ou de la chambre **10** de l'autoclave **1.**

### Autoclave

Un autoclave **1** pour la mise en oeuvre du procédé décrit ci-dessus est décrit ci-après en référence à la figure 2.

L'autoclave **1** comprend une chambre **10** pour recevoir un mélange aqueux à haute température et un échantillon **2** destiné à être analysé pour l'étude des interactions du matériau composant l'échantillon avec de l'hydrogène du mélange aqueux à haute température.

Pour l'introduction du mélange aqueux à haute température, l'autoclave **1** comprend un circuit de recirculation comprenant une vanne d'entrée **15** et une vanne de sortie **16.**

Afin de refroidir l'échantillon **2** et/ou la chambre **10** de l'autoclave **1** à la fin de l'exposition de l'échantillon **2** avec le mélange aqueux, l'autoclave **1** comprend un injecteur haute pression **17.** Cet injecteur haute pression **17** est adapté pour injecter du liquide de refroidissement sous une haute pression supérieure à 50 bars, et de préférence supérieure à 70 bars.

L'injecteur haute pression **17** peut comprendre un clapet anti-retour **12** haute pression. Ce clapet anti-retour **12** haute pression s'ouvre automatiquement sous l'effet du différentiel de pression pour faire passer le liquide de refroidissement sous haute pression dans la chambre **10** dès que la chambre **10** est sous une pression inférieure à la haute pression. Le clapet anti-retour **12** est relié à la chambre **10** de l'autoclave **1** par un conduit **19.**

Le liquide de refroidissement peut être mis sous haute pression à l'aide d'une bouteille de gaz neutre, par exemple du diazote ou de l'argon comme connu de l'homme du métier. Ces exemples ne sont pas limitatifs, il pourrait s'agir par exemple d'huile mise sous haute pression à l'aide d'un autre gaz neutre comme l'hélium par exemple.

L'autoclave **1** peut également comprendre un échappement **14.** Cet échappement **14** est ouvrable pour l'évacuation du liquide aqueux du mélange hors de la chambre **10.** L'évacuation du liquide aqueux provoque une détente de la vapeur du mélange à l'intérieur de la chambre **10** de l'autoclave **1.**

L'autoclave **1** peut aussi comprendre un injecteur basse pression **18** pour l'injection de liquide de refroidissement sous une basse pression comprise entre 2 et 11 bars dans la chambre **10** de l'autoclave **1.**

Le liquide de refroidissement sous basse pression peut provenir directement des canalisations d'eau courante.

L'injecteur basse pression **18** peut comprendre un clapet anti-retour **13** basse pression. Ce clapet anti-retour **13** basse pression s'ouvre pour faire passer le liquide de refroidissement sous basse pression dans la chambre d'exposition **10** dès que la chambre d'exposition **10** est sous une pression inférieure à la basse pression. Un conduit **20** relie le clapet anti-retour **13** basse pression à la chambre **10** de l'autoclave **1.**

### Exemple de dimensionnement et résultats

La chambre **10** peut être formée par un tube ayant un diamètre compris entre 15 et 35 mm et une épaisseur de paroi comprise entre 1,5 et 5 mm.

Pour une chambre **10** ayant les dimensions mentionnées ci-dessus, l'étape d'injection **E3** de liquide de refroidissement sous haute pression dure environ 30 secondes puis l'étape d'injection **E4** de liquide de refroidissement sous basse pression dure environ 3 minutes. La durée de refroidissement totale est donc inférieure à environ 4 minutes.

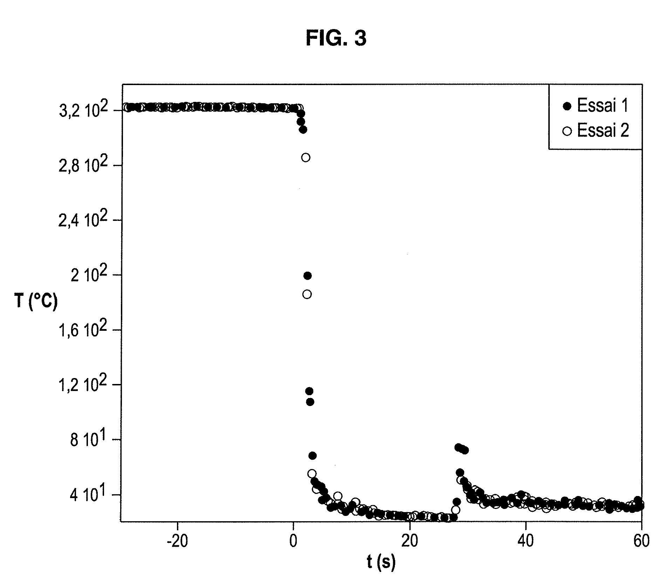

En réalité, l'échantillon **2** est déjà à température ambiante, environ 20° C moins de 10 secondes après l'ouverture de l'échappement **14** comme le montre la figure 3. Cependant, la chambre **10** n'est pas encore suffisamment refroidie pour pouvoir être ouverte et extraire l'échantillon **2.** Le maintien de l'étape d'injection **E3** de liquide de refroidissement sous haute pression pendant 30 secondes environ est nécessaire pour éviter une remontée significative en température de l'échantillon **2** due à l'échange thermique entre la chambre **10** et l'échantillon **2.**

Ensuite, la substitution du liquide de refroidissement sous haute pression par le liquide de refroidissement sous basse pression permet d'économiser sur le coût d'utilisation du procédé par rapport à un procédé utilisant uniquement une injection de liquide de refroidissement sous haute pression (qui nécessite par exemple une bouteille de gaz comprimée).

Un avantage du procédé et de l'autoclave pour la mise en oeuvre du procédé selon l'invention est qu'il est possible de diminuer le temps de refroidissement nécessaire pour que l'échantillon atteigne une température avoisinant les 50°C. Ce temps de refroidissement est amené de plusieurs heures à quelques minutes.

Puisque le temps de refroidissement est relativement court, l'hydrogène absorbé par le matériau constituant l'échantillon n'a pas le temps de désorber. L'état du matériau constituant l'échantillon à la fin du procédé selon l'invention représente donc plus fidèlement l'état du matériau constituant l'échantillon quand celui-ci est au contact du mélange aqueux haute température.

## Revendications

1. Procédé de préparation d'un échantillon (2) métallique introduit dans une chambre d'exposition (10) d'un autoclave (1), l'échantillon (2) étant destiné à être analysé pour l'étude des interactions du matériau composant l'échantillon (2) avec de l'hydrogène d'un mélange aqueux à haute température auquel l'échantillon (2) est exposé dans la chambre (10), **caractérisé en ce qu'**il comprend une étape de refroidissement par injection (E3) de liquide de refroidissement sous une haute pression supérieure à 50 bars dans la chambre d'exposition de l'autoclave par l'intermédiaire d'un injecteur (17), pour refroidir l'échantillon (2) et la chambre d'exposition (10) et ainsi éviter une évaporation du liquide de refroidissement sous haute pression lors de son contact avec l'échantillon (2) et/ou la chambre d'exposition (10).

2. Procédé selon la revendication 1, dans lequel le liquide de refroidissement sous haute pression est injecté sous une pression supérieure à 70 bars.

3. Procédé selon la revendication 1 ou 2, comprenant en outre une étape d'ouverture (E2) d'un échappement (14) de l'autoclave (1), antérieure à l'étape de refroidissement par injection (E3) de liquide de refroidissement sous haute pression, pour l'évacuation du liquide aqueux du mélange hors de la chambre, provoquant une détente de la vapeur du mélange à l'intérieur de la chambre (10) de l'autoclave (1) et ainsi refroidissant l'échantillon et/ou la chambre (10) de l'autoclave (1).

4. Procédé selon l'une des revendications 1 à 3, comprenant en outre l'étape d'injection (E4) de liquide de refroidissement sous une basse pression comprise entre 2 et 11 bars dans l'autoclave (1) pour maintenir relativement constante la température de l'échantillon (2) et continuer le refroidissement de la chambre (10) de l'autoclave (1), l'étape d'injection (E4) de liquide de refroidissement sous basse pression étant postérieure à l'étape d'injection (E3) de liquide de refroidissement sous haute pression.

5. Procédé selon la revendication 4, dans lequel l'étape d'injection (E3) de liquide de refroidissement sous haute pression est arrêtée avant l'étape d'injection (E4) de liquide de refroidissement sous basse pression.

6. Autoclave (1) pour la mise en oeuvre du procédé selon l'une des revendications 1 à 5, comprenant une chambre d'exposition (10) pour recevoir un mélange aqueux à haute température et un échantillon (2) destiné à être analysé pour l'étude des interactions du matériau composant l'échantillon avec de l'hydrogène du mélange aqueux à haute température ;
**caractérisé en ce qu'**il comprend, en outre, un injecteur haute pression (17) pour l'injection de liquide de refroidissement sous une haute pression supérieure à 50 bars.

7. Autoclave (1) selon la revendication 6, dans lequel l'injecteur haute pression (17) comprend un clapet anti-retour (12) haute pression ouvrable pour faire passer le liquide de refroidissement sous haute pression dans la chambre (10) quand la chambre (10) est sous une pression inférieure à la haute pression.

8. Autoclave (1) selon la revendication 6 ou 7, comprenant un échappement (14) ouvrable pour l'évacuation du liquide aqueux du mélange hors de la chambre, provoquant une détente de la vapeur du mélange à l'intérieur de la chambre (10) de l'autoclave (1).

9. Autoclave (1) selon l'une des revendications 6 à 8, comprenant en outre un injecteur basse pression (18) pour l'injection de liquide de refroidissement sous une basse pression comprise entre 2 et 11 bars dans la chambre (10) de l'autoclave (1).

10. Autoclave (1) selon l'une des revendications 6 à 9, dans lequel l'injecteur basse pression (18) comprend un clapet anti-retour (13) basse pression ouvrable pour faire passer le liquide de refroidissement sous basse pression dans la chambre d'exposition (10) quand la chambre d'exposition (10) est sous une pression inférieure à la basse pression.

## Patentansprüche

1. Verfahren zur Vorbereitung einer metallischen Probe (2), die in eine Expositionskammer (10) eines Autoklaven (1) eingeführt wird, wobei die Probe (2) dazu bestimmt ist, für die Untersuchung der Interaktionen des Materials, aus dem die Probe (2) besteht, mit Wasserstoff eines wässrigen Gemisches bei hoher Temperatur analysiert zu werden, dem die Probe (2) in der Kammer (10) ausgesetzt wird, **dadurch gekennzeichnet, dass** es einen Schritt der Abkühlung durch Einspritzen (E3) von Kühlflüssigkeit unter einem hohen Druck von über 50 Bar in die Expositionskammer des Autoklaven über eine Einspritzdüse (17) umfasst, um die Probe (2) und die Expositionskammer (10) abzukühlen und so eine Verdampfung der Kühlflüssigkeit unter hohem Druck bei ihrem Kontakt mit der Probe (2) und/oder der Expositionskammer (10) zu vermeiden.

2. Verfahren nach Anspruch 1, wobei die Kühlflüssigkeit unter hohem Druck mit einem Druck eingespritzt wird, der höher als 70 Bar ist.

3. Verfahren nach Anspruch 1 oder 2, das ferner einen Schritt der Öffnung (E2) eines Auslasses (14) des Autoklaven (1) vor dem Schritt der Abkühlung durch Einspritzung (E3) von Kühlflüssigkeit unter hohem Druck für den Ablass der wässrigen Flüssigkeit des Gemisches aus der Kammer umfasst, wodurch ein Druckabfall des Dampfes des Gemischs im Inneren der Kammer (10) des Autoklaven (1) und somit die Abkühlung der Probe und/oder der Kammer (10) des Autoklaven (1) bewirkt wird.

4. Verfahren nach einem der Ansprüche 1 bis 3, das ferner den Schritt des Einspritzens (E4) von Kühlflüssigkeit unter einem niedrigen Druck, der zwischen 2 und 11 Bar enthalten ist, in den Autoklaven (1) umfasst, um die Temperatur der Probe (2) relativ konstant zu halten und die Abkühlung der Kammer (10) des Autoklaven (1) fortzusetzen, wobei der Schritt der Einspritzung (E4) der Kühlflüssigkeit unter niedrigem Druck nach dem Schritt der Einspritzung (E3) von Kühlflüssigkeit unter hohem Druck erfolgt.

5. Verfahren nach Anspruch 4, wobei der Schritt der Einspritzung (E3) von Kühlflüssigkeit unter hohem Druck vor dem Schritt der Einspritzung (E4) von Kühlflüssigkeit unter niedrigem Druck angehalten wird.

6. Autoklav (1) für die Umsetzung des Verfahrens nach einem der Ansprüche 1 bis 5, der eine Expositionskammer (10) zum Aufnehmen eines wässrigen Gemischs mit hoher Temperatur und einer Probe (2) umfasst, die dazu bestimmt ist, für die Untersuchung der Interaktionen des Materials, aus dem die Probe besteht, mit Wasserstoff des wässrigen Gemisches bei hoher Temperatur analysiert zu werden;
**dadurch gekennzeichnet, dass** es ferner eine Hochdruck-Einspritzdüse (17) für die Einspritzung von Kühlflüssigkeit unter einem hohen Druck von über 50 Bar umfasst.

7. Autoklav (1) nach Anspruch 6, wobei die Hochdruck-Einspritzdüse (17) ein Hochdruck-Rückschlagventil (12) umfasst, das betriebsfähig ist, um die unter hohem Druck stehende Kühlflüssigkeit in die Kammer (10) durchzulassen, wenn die Kammer (10) unter einem Druck steht, der niedriger als der hohe Druck ist.

8. Autoklav (1) nach Anspruch 6 oder 7, der einen Auslass (14) umfasst, der für den Ablass der wässrigen Flüssigkeit des Gemisches aus der Kammer betriebsfähig ist, wodurch ein Druckabfall des Dampfes der Mischung im Inneren der Kammer (10) des Autoklaven (1) bewirkt wird.

9. Autoklav (1) nach einem der Ansprüche 6 bis 8, der ferner eine Niederdruck-Einspritzdüse (18) für die Einspritzung von Kühlflüssigkeit unter einem niedrigen Druck, der zwischen 2 und 11 Bar enthalten ist, in der Kammer (10) des Autoklaven (1) umfasst.

10. Autoklav (1) nach einem der Ansprüche 6 bis 9, wobei die Niederdruck-Einspritzdüse (18) ein Niederdruck-Rückschlagventil (13) umfasst, das betriebsfähig ist, um die Kühlflüssigkeit unter niedrigem Druck in die Expositionskammer (10) durchzulassen, wenn die Expositionskammer (10) unter einem Druck steht, der niedriger ist als der niedrige Druck.

## Claims

1. A method for preparing a metal sample (2) introduced into an exposure chamber (10) of an autoclave (1), the sample (2) being intended to be analyzed for studying the interactions of the material composing the sample (2) with hydrogen of an aqueous mixture at a high temperature to which the sample (2) is exposed in the chamber (10), **characterized in that** it comprises a step of cooling by injection (E3) of cooling liquid under a high pressure greater than 50 bars into the exposure chamber of the autoclave by means of an injector (17), to cool the sample (2) and the exposure chamber (10) and to thus avoid evaporation of the high pressure cooling liquid during its contact with the sample (2) and/or the exposure chamber (10).

2. The method according to claim 1, wherein the high pressure cooling liquid is injected under a pressure greater than 70 bar.

3. The method according to claim 1 or 2, further comprising a step of opening (E2) an exhaust (14) of the autoclave (1), prior to the step of cooling by injection (E3) of cooling liquid under high pressure, to remove the aqueous liquid of the mixture from the chamber, causing an expansion of the vapor of the mixture within the chamber (10) of the autoclave (1) and thus cooling the sample and/or the chamber (10) of the autoclave (1).

4. The method according to one of claims 1 to 3, further comprising the step of injecting (E4) cooling liquid under low pressure comprised between 2 and 11 bars into the autoclave (1) to hold the temperature of the sample (2) relatively constant and continue the cooling of the chamber (10) of the autoclave (1), the step of injecting (E4) cooling liquid under low pressure occurring after the step of injecting (E3) cooling liquid under high pressure.

5. The method according to claim 4, wherein the step of injecting (E3) cooling liquid under high pressure is stopped prior to the step of injecting (E4) cooling liquid under low pressure.

6. An autoclave (1) for implementing the method according to one of claims 1 to 5, comprising an exposure chamber (10) to receive an aqueous mixture at high temperature and a sample (2) intended to be analyzed for studying the interactions of the material composing the sample with hydrogen of the aqueous mixture at high temperature;
**characterized in that** it further comprises a high pressure injector (17) for injecting cooling liquid under high pressure greater than 50 bars.

7. The autoclave (1) according to claim 6, wherein the high pressure injector (17) comprises a high pressure check valve (12) which can be opened to allow the high pressure cooling liquid to pass into the chamber (10) when the chamber (10) is under a pressure less than the high pressure.

8. The autoclave (1) according to claim 6 or 7, comprising an exhaust (14) which can be opened to remove the aqueous liquid of the mixture from the chamber, causing an expansion of the vapor of the mixture within the chamber (10) of the autoclave (1).

9. The autoclave (1) according to one of claims 6 to 8, further comprising a low-pressure injector (18) for injecting cooling liquid under low pressure comprised between 2 and 11 bars into the chamber (10) of the autoclave (1).

10. The autoclave (1) according to one of claims 6 to 9, wherein the low pressure injector (18) comprises a low pressure check valve (13) that can be opened to allow the low pressure cooling liquid to pass into the exposure chamber (10) when the exposure chamber (10) is at a pressure less than the low pressure.
